(19) Europäisches Patentamt
European Patent Office
Office européen des brevets

(11) Veröffentlichungsnummer: **0 007 080**
**B1**

# (12) EUROPÄISCHE PATENTSCHRIFT

(45) Veröffentlichungstag der Patentschrift: **29.07.81**

(21) Anmeldenummer: **79102314.6**

(22) Anmeldetag: **09.07.79**

(51) Int. Cl.³: **C 07 D233/68**, C 07 D231/12, C 07 D231/18, C 07 D249/08, C 07 C103/375

## (54) Verfahren zur Herstellung von N-azolylmethyl-substituierten Halogenacetaniliden.

(30) Priorität: **13.07.78 DE 2830764**

(43) Veröffentlichungstag der Anmeldung: **23.01.80 (Patentblatt 80/02)**

(45) Bekanntmachung des Hinweises auf die Patenterteilung: **29.07.81 Patentblatt 81/30**

(84) Benannte Vertragsstaaten: **AT BE CH DE FR GB IT LU NL SE**

(56) Entgegenhaltungen:
**DE - A - 1 542 956**
**DE - A - 2 648 008**
**DE - A - 2 744 396**
**DE - A - 2 704 281**
**US - A - 3 442 945**
**US - A - 3 637 847**

(73) Patentinhaber: **BASF Aktiengesellschaft**
**Carl-Bosch-Strasse 38**
**D-6700 Ludwigshafen (DE)**

(72) Erfinder: **Eicken, Karl, Dr. Chem.**
**Waldstrasse 63**
**D-6706 Wachenheim (DE)**
Erfinder: **Rohr, Wolfgang, Dr. Chem.**
**Gontardstrasse 4**
**D-6800 Mannheim 1 (DE)**
Erfinder: **Linhart, Friedrich, Dr. Chem.**
**Leisberg 61**
**D-6900 Heidelberg (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 17, rue du Louvre, 75001 Paris, France

EP 0 007 080 B1

Verfahren zur Herstellung von N-azolylmethylsubstituierten Halogenacetaniliden

Die vorliegende Erfindung betrifft ein Verfahren zur Herstellung von Acetaniliden durch Umsetzung von 2-Halogen-N-halogenmethylacetaniliden mit Azolen in einem Zweiphasensystem, gegebenenfalls unter Verwendung eines Phasentransferkatalysators.

Es ist bekannt (US-PS 3 637 847 (S. 5, Zeile 14) und DE-OS 1 542 956 (S. 5 u. 6)), daß die Halogenatome in der N-Halogenmethylgruppe von 2-Halogen-N-halogenmethylacetaniliden sehr reaktiv sind und mit Wasser bereits bei Raumtemperatur (20 °C) und darunter hydrolysieren.

Es ist ferner bekannt (US-PS 3 442 945 u. DE-AS 1 543 751), Umsetzungen solcher 2-Halogen-N-halogen-methylacetanilide, z. B. mit Alkoholen, unter Ausschluß von Feuchtigkeit und Verwendung wasserfreier Reaktionsteilnehmer durchzuführen.

Es ist ferner bekannt (DE-OS 2 648 008, DE-OS 2 744 396), Acetanilide mit N-Methyl-Azolresten unter Einhaltung wasserfreier Bedingungen aus 2-Halogen-N-halogenmethylacetaniliden und Azolen herzustellen. Beispielsweise werden dabei Alkalisalze der Azole unter wasserfreien Bedingungen hergestellt und mit 2-Halogen-halogenmethylacetaniliden unter Ausschluß von Feuchtigkeit umgesetzt. Diese bekannte Verfahrensweise ist aufwendig und für eine technische Herstellung wenig geeignet.

Es wurde nun gefunden, daß man Acetanilide der allgemeinen Formel I

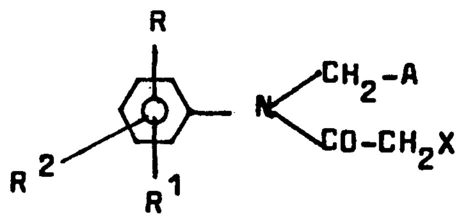

(I)

worin R Wasserstoff, Alkyl bis zu 5-C-Atomen, Alkoxy bis zu 5-C-Atomen ;

$R^1$ Wasserstoff, Halogen bis zu 5-C-Atomen, Alkoxy bis zu 5-C-Atomen oder Perhalogenalkyl bis zu 5 Kohlenstoffatomen,

$R^2$ Wasserstoff, Halogen, Alkyl bis zu 5-C-Atomen, Alkoxy bis zu 5-C-Atomen bedeutet oder $R^2$ zusammen mit R eine orthoständig verknüpfte, gegebenenfalls durch Alkylgruppen mit bis zu 4-C-Atomen substituierte Alkylenkette mit bis zu 6-Kohlenstoffatomen bedeutet,

X Chlor oder Brom,

A einen über ein Ringstickstoffatom gebundenen Azolyl-Rest bedeutet, der einfach oder mehrfach durch Halogen, Phenylreste, Alkyl, Alkoxy, Alkylthio, Perfluoralkylreste mit jeweils bis zu 4-C-Atomen, Cyan oder Carboalkoxyresten mit bis zu 4-C-Atomen substituiert sein kann, in einfacher Weise erhält, wenn man 2-Halogen-N-halogenmethylacetanilide der allgemeinen Formel II

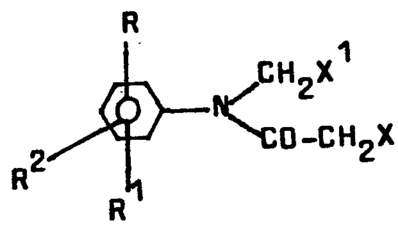

(II)

in welcher R, $R^1$, $R^2$ und X die oben genannten Bedeutungen haben und $X^1$ die gleichen Bedeutungen wie X hat, wobei X und $X^1$ im Einzelfall gleich oder verschieden sind, mit mindestens molaren Mengen eines Azols der Formal H-A, in der A die oben genannten Bedeutungen hat und mindestens molaren Mengen wäßriger Alkalien in einem Zweiphasensystem, gegebenenfalls in Gegenwart eines Phasentransferkatalysators, umsetzt.

Einige der als Ausgangsstoffe verwendeten 2-Halogen-N-halogenmethylacetanilide der allgemeinen Formel II sind bekannt aus der DE-AS 1 542 956. Andere können auf analoge Weise durch Umsetzung der entsprechenden Azomethine mit einem Halogenacetyl-halogenid hergestellt werden.

Das 1-H-Azol der allgemeinen Formel H-A wird zweckmäßig in mindestens molarer Menge bezogen auf das 2-Halogen-N-halogenmethylacetanilid, maximal in bis zu ein-molarem Überschuß, verwendet.

Als 1-H-Azole der allgemeinen Formel H-A werden vorzugsweise die Verbindungen 2,6-Dimethylpyrrol, Tetramethylpyrrol, 3(5)-Methylpyrazol, 4-Methylpyrazol, 3(5)-Äthylpyrazol, 4-Äthylpyrazol, 3(5)-Isopropylpyrazol, 4-Isopropylpyrazol, 3,5-Dimethylpyrazol, 3,5-Dimethyl-4-acetylpyrazol, 3,5-Dimethyl-4-propionylpyrazol, 3,4,5-Trimethylpyrazol, 3(5)-Phenylpyrazol, 4-Phenylpyrazol, 3,5-Diphenylpyrazol, 3(5)-phenyl-5(3)-methylpyrazol, 3(5)-Chlorpyrazol, 4-Chlorpyrazol, 4-Brompyrazol, 4-Jodpyrazol, 3,4,5-Trichlorpyrazol, 3,4,5-Tribrompyrazol, 3,5-Dimethyl-4-chlorpyrazol, 3,5-Dimethyl-4-brompyrazol, 4-Chlor-3(5)-methylpyrazol, 4-Brom-3(5)-methylpyrazol, 4-Methyl-3,5-dichlorpyrazol, 3(5)-Methyl-4,5(3)-dichlorpyrazol, 3(5)-Chlor-(5)3-methylpyrazol, 4-Methoxypyrazol, 3(5)-Methyl-5(3)-methoxypyrazol, 3(5)-Äthoxy-4,5(3)-dimethylpyrazol, 3(5)-Methyl-5(3)-trifluormethylpyrazol, 3,5-Bistrifluormethylpyrazol, 3(5)-Methyl-5(3)-carbäthoxypyrazol, 3,5-Biscarbäthoxypyrazol, 3,4,5-Triscarbäthoxypyrazol, 3(5)-Methyl-5(3)-methylthio-4-carbäthoxypyrazol, 4-Methyl-3,5-biscarbäthoxypyrazol, 4-Cyanopyrazol, 4-Methoxy-3,5-dichlorpyrazol, 3(5)-Methyl-1,2,4-triazol, 3,5-Dimethyl-1,2,4-triazol, 3(5)-Chlor-1,2,4-triazol, 3(5)-Brom-1,2,4-triazol, 3(5)-Chlor-5(3)-methyl-1,2,4-triazol, 3,5-Dichlor-1,24-triazol, 3,5-Dibrom-1,2,4-triazol, 3,5-Dibrom-1,2,4-triazol, 3(5)-Chlor-5(3)-cyano-1,2,4-triazol, 3(5)-Chlor-5(3)-phenyl-1,2,4-triazol, 3(5)-Chlor-5(3)-carbomethoxy-1,2,4-triazol, 3(5)-Methylthio-1,2,4-triazol, 4(5)-Methyl-1,2,3-triazol, 4,5-Dimethyl-1,2,3-triazol, 4(5)-Phenyl-1,2,3-triazol, 4(5)-Chlor-1,2,3-triazol, 1,2,3-Triazol-4(5)-yl-carbonsäureäthylester, 1,2,3-Triazol-4,5-yl-dicarbonsäuredimethylester, 5-Methyltetrazol, 5-Chlortetrazol, Tetrazolyl-5-carbonsäureäthylester, Imidazol, 2-Methylimidazol, 4(5)-Chlorimidazol, 2-Chlorimidazol, 4,5-Dichlorimidazol, Trichlorimidazol, 2-Methyl-4,5-dichlorimidazol, 2-Äthyl-4,5-dichlorimidazol, 2-Isopropyl-4,5-dichlorimidazol, 4(5)-Bromimidazol, 4,5-Dibromimidazol, Tribomimidazol, 2-Methyl-4,5-dibromimidazol, 2-Brom-4,5-dichlorimidazol verwendet. Ein Zweiphasensystem ist eine Mischung aus Wasser und einem organischen Lösungsmittel, das mit Wasser nicht mischbar ist. Die Flüssigkeiten enthalten chemische Verbindungen gelöst oder in Form einer Dispersion.

Der bei der erfindungsgemäßen Umsetzung im Zweiphasensystem freiwerdende Halogenwasserstoff $HX^1$ wird durch wäßrige Alkalien z. B. Natronlauge, Kalilauge, Sodalösung, Kaliumcarbonatlösung, Kalkmilch (Lösung oder Dispersion von $Ca(OH)_2$) gebunden. Die wäßringen Alkalien werden mindestens in molarer Menge, beispielsweise bis zu maximal zwei-molarem Überschuß bezogen auf 2-Halogen-N-halogenmethylacetanilid verwendet.

Als organische Phase zur Durchführung der Zweiphasenreaktion eignen sich unter den Reaktionsbedingungen inerte, organische Lösungsmittel, die nicht mit der wäßrigen Phase mischbar sind, beispielsweise aliphatische oder aromatische Kohlenwasserstoffe, z. B. Ligroin, Cyclohexan, Benzol, Toluol, Xylole ; beispielsweise halogenierte aliphatische oder aromatische Kohlenwasserstoffe, z. B. Methylenchlorid, Chloroform, Tetrachlorkohlenstoff, Chlorbenzol, Dichlorbenzole ;

beispielsweise Äther, z. B. Diäthyläther, Diisopropyläther, Anisol, Phenetol ; beispielsweise Ester, z. B. Essigester, Butylacetat.

Die bei der Durchführung der Umsetzung einzuhaltenden Temperaturen liegen beispielsweise zwischen − 30° bis + 100 °C, vorzugsweise bei − 10 bis + 50 °C, insbesondere bei Raumtemperatur (20 °C). Die Umsetzung wird beispielsweise bei Normaldruck (1 bar) durchgeführt.

Zur Erzielung einer guten Ausbeute an Endprodukten und einer guten Reinheit der Endprodukte ist es zweckmäßig, die Hydrolyse des Ausgangsproduktes 2-Halogen-N-halogenmethylacetanilid mit Wasser durch Zusatz einer als Phasentransferkatalysator geeigneten Verbindung aus der Gruppe der Onium-Salze, nämlich der quaternären Ammonium-, Phosphonium- oder Sulfoniumsalze oder der entsprechenden Hydroxide, die auch an feste Träger gebunden sein können, oder der makrocyclischen Heterocyclen, vorzugsweise makrocyclischen Polyäther oder Polyäthylenglykol-diäther oder oberflächenaktiven Substanzen, in einer Menge von 0,01-30 Molprozent bezogen auf das 2-Halogen-N-halogenmethylacetanilid zu vermeiden.

Als Phasentransferkatalysator kommen « Onium »-Verbindungen in Frage, beispielsweise quaternäre Ammoniumverbindungen, z.B. Tetrabutylammoniumchlorid, Tetrabutylammoniumbromid, Tetrabutylammoniumhydrogensulfat, Tetrapentylammoniumchlorid, Tetraoctylammoniumchlorid, Tripropylbutylammoniumchlorid, Tricaprylyl-methylammoniumchlorid, Hexadecyl-trimethylammoniumchlorid, Distearyl-dimethylammoniumchlorid, Dibenzyldimethylammonium-methylsulfat, Dimethyldodecyl-benzylammoniumchlorid, Benzyltrimethylammoniumchlorid, Benzyl-triäthylammoniumchlorid, 2-Hydroxyäthyltrimethylammoniumchlorid ; beispielsweise quaternäre Phosphoniumverbindungen z. B. Tributylmethylphosphoniumbromid, Hexadecyl-tributylphosphoniumbromid, Äthyl-triphenyl-phosphoniumbromid, Tetraphenylphosphoniumbromid oder die entsprechenden Hydroxide dieser Onium-Verbindungen.

Die Onium-Verbindungen können auch an feste Träger gebunden sein (Synthesis 1978, 315, J. org. Chem. 42 (1977) s. 875-879).

Ferner können als Phasentransferkatalysatoren makrocyclische Heterocyclen, vorzugsweise makrocyclische Polyäther, verwendet werden, wie sie in Synthesis *1976*, s. 168-188, beschrieben werden. Z. B. 15-Krone-5, 18-Krone-6, Dibenzo-18-krone-6, Dicyclohexyl-18-krone-6 (Kronenäther).

Ferner sind als Phasentransfer-Katalysatoren Polyäthylenglykol-dialkyläther bestimmter Kettenlänge (DE-OS 2 534 851 bzw. Synthesis *1977*, S. 184-186) für die erfindungsgemäße Durchführung des Verfahrens geeignet. Andere geeignete Phasentransferkatalysatoren sind oberflächenaktive Mittel (Tenside) nach der Definition in Kirk-Othmer, « Encyclopedia of Chemical Technology », 2. Ausgabe, Bd. 19 (1969), S. 508. Die oberflächenaktiven Substanzen sind vorzugsweise nicht-ionisch, z. B. Polyl (alkylenoxy) derivate. Sie können durch Umsetzung von höheren Alkoholen oder Alkylphenolen oder Fettsäuren mit Äthylenoxid oder Propylenoxid mit 1 bis 50 Einheiten Alkylenoxid je Mol Alkohol, Phenol oder Säure hergestellt werden. Die Konzentration des Azols in der wäßrigen Phase beträgt beispielsweise 5 bei 60 Gew. %. Die Konzentration des Alkalis in der wäßrigen Phase beträgt beispielsweise 5 bei 30 Gew. %. Die Konzentration des 2-Halogen-N-halogenmethylacetanilids in der organischen Phase beträgt beispielsweise 5 bis 40 Gew. %.

Zweckmäßig führt man die Umsetzung unter intensiver Durchmischung der zwei Phasen beispielsweise unter starken Rühren durch. Für die Durchführung des erfindungsgemäßen Verfahrens ist es unerheblich, ob man im Umsetzungsgefäß die organische Phase, enthaltend das 2-Halogen-N-halogenmethylacetanilid, vorlegt und die wäßrige Phase enthaltend das Azol und das wäßrige Alkali unter intensiver Durchmischung zudosiert oder aber die genannte wäßrige Phase vorlegt und die organische Phase zudosiert (Diskontinuierliches Verfahren). Ebenso können beide Phasen gleichzeitig aus zwei Dosiervorrichtungen in vorgelegtes, inertes organisches Lösungsmittel oder eine ungereinigte Lösung von technischem Azolyl-Acetanilid zugesetzt werden, wobei die Durchmischung der zwei Phasen in einem Mischkreis oder einer Mischstrecke erfolgen kann. Nach Abtrennung der wäßrigen Phase, die im wesentlichen, insbesondere bei Verwendung stöchiometrischer Mengen an Ausgangsprodukten eine Alkalihalogenid-Lösung ist und nach Abtrennung eines Teils der organischen Phase kann der Rest der organischen Phase in den Mischkreis zurückgeführt werden (Kontinuierliches Verfahren).

Der Phasentransferkatalysator kann wahlweise der organischen oder der wäßrig-alkalischen Phase zugesetzt werden, wobei es bei diskontinuierlichem Arbeiten vorteilhaft ist, den Phasentransfer-Katalysator der im Umsetzungsgefäß vorgelegten Phase zuzusetzen.

Verwendet man als Ausgangsprodukte unsymmetrisch substituierte Azole, z. B. mono-, di- oder trisubstituierte Pyrazole oder Imidazole oder 1,2,3-Triazole oder 1,2,4-Triazole, so treten aufgrund tautomerer Strukturen in den Ausgangsstoffen der allg. Formel H-A bei der erfindungsgemäßen Herstellung der Acetanilide der allg. Formel I auch zwei Isomere der folgenden Formeln auf, deren Isomerenverhältnis im wesentlichen von der Art der Reste (B, C, D im Pyrazol z. B.) bestimmt wird.

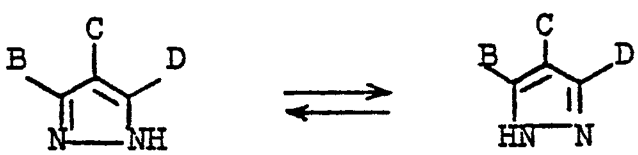

Die folgenden Beispiele erläutern das erfindungsgemäße Verfahren, ohne es einzuschräken. Hierbei verhalten sich Raumteile zu Gewichtsteilen wie Liter zu Kilogramm.

Beispiel 1

Zu einer Lösung von 24,6 Gew. Teilen 2-Chlor-N-chlormethyl-2',6'-dimethylacetanilid und 2,0 Gew. Teilen Triäthylbenzyl-ammoniumchlorid in 50 Vol.-Teilen Dichlormethan wurde bei 20 bis 25 °C eine Lösung von 13,7 Gew.-Teilen 4,5-Dichlorimidazol und 4,4 Gew. Teilen Natriumhydroxid in 20 Teilen Wasser unter Turbinieren (starkem Rühren) zugetropft und weitere 2 Std. turbiniert. Die organische Phase lieferte nach dreimaligem Waschen mit je 30 Vol.-Teilen Wasser, Trocknen über Natriumsulfat und Eindampfen im Vacuum bei 70 °C 29,9 Gew. Teile 2-Chlor-2',6'-dimethyl-N-(4,5-dichlorimidazol-1-yl-methyl)-acetanilid vom Fp 99 °C, die nach $^{1}$H-NMR-Spektrum und DC (Dünnschichtchromatographie) (Essigester : Toluol 3 : 7) rein waren.

Beispiel 2

Zu einer Lösung von 24,6 Gew. Teilen 2-Chlor-N-chlormethyl-2',6'-dimethylacetanilid und 0,15 Gew. Teilen Tri-n-butylmethylphosphoniumbromid in 80 Vol. Teilen o-Dichlorbenzol wurde bei 0 °C eine Lösung von 9,0 Gew. Teilen 4-Methylpyrazol und 4,0 Gew. Teilen Natriumhydroxid in 20 Vol.-Teilen Wasser unter Turbinieren zugetropft und 5 Std. bei 10 °C turbiniert. Die organische Phase ergab nach dreimaligem Waschen mit je 50 Vol. Teilen Wasser, Trocknen über Natriumsulfat und Eindampfen i. Vac. bei 70 °C 25,6 Gew. Teile 2-Chlor-2',6'-dimethyl-N-(4-methylpyrazol-1-yl-methyl)-acetanilide vom Fp.: 100 °C, die nach $^{1}$H-NMR und DC (Essigester : Toluol/3 : 7) rein waren.

Beispiel 3

Zu einer Lösung von 24,6 Gew.-Teilen 2-Chlor-N-chlormethyl-2',6'-dimethylacetanilid und einem Gew. Teil Benzyltriäthylammoniumbromid in 70 Vol. Teilen Toluol wurden bei 10-15 °C eine Lsg. von 10,8 Gew. Teilen 4-Methoxypyrazol und 4,0 Gew. Teilen Natriumhydroxid in 20 Vol. Teilen Wasser unter Turbinieren zugetropft und 3 Std. weiter turbiniert. Die organ. Phase lieferte nach dreimaligem Waschen mit je 40 Vol. Teilen Wasser, Trocknen über Natriumsulfat und Eindampfen i. Vac. bei 80 °C 28,1 Gew. Teile 2-Chlor-2',6'-dimethyl-N-(4-methoxypyrazol-1-yl-methyl)-acetanilid vom Fp. 115-116 °C (Probe aus Äthanol 118 °C), die nach 1H-NMR und DC (Essigester : Toluol/3 : 7) über 97 %ig waren.

Beispiel 4

Zu einer Lösung von 24,6 Gew. Teilen 2-Chlor-N-chlormethyl-2',6-dimethylacetanilid und 0,5 Gew. Teilen Triäthylbenzylammoniumchlorid in 100 Vol. Teilen Chloroform wurde bei 25 °C eine Lösung von

12,1 Gew. Teilen 4-Isopropylpyrazol und 5,6 Gew. Teilen Kaliumhydroxid in 30 Vol. Teilen Wasser unter Turbinieren zudosiert und 4 Std. bei 25 °C weiter turbiniert. Die organische Phase lieferte nach dreimaligem Waschen mit je 50 Vol.-Teilen Wasser, Trocknen über Natriumsulfat und Eindampfen i. Vac. bei 50 °C 26,2 Gew. Teile 2-Chlor-2',6'-dimethyl-N-(4-isopropylpyrazol-1-yl-methyl)-acetanilid vom Fp. 58 °C.

## Beispiel 5

Zu einer Lösung von 49,2 Gew. Teilen 2-Chlor-N-chlormethyl-2',6'-dimethylacetanilid und 6,2 Gew. Teilen Tetrabutylammoniumhydrogensulfat in 200 Vol. Teilen Methylenchlorid wurde eine Lösung von 27,4 Gew. Teilen 1,2,4-Triazol und 11,2 Gew. Teilen Kaliumhydroxid in 50 Vol. Teilen Wasser bei Raumtemperatur zugetropft und 5 Std. turbiniert. Aus der organischen Phase erhielt man nach dreimaligem Waschen mit je 80 Vol.-Teilen Wasser, Trocknen über Natriumsulfat und Verdampfen des Lösungsmittels 1. Vac. bei 60 °C 46,1 Gew. Teile einer Kristallmasse, die zu 88 % aus 2-Chlor-2',6'-dimethyl-N-(1,2,4-triazol-1-yl-methyl) acetanilid vom Fp. 115-118 °C und zu 10 % aus 2-Chlor-2',6'-dimethylacetanilid vom Fp. 140-143 °C bestanden (säulenchromatographische Trennung an Kieselgel mit Methylenchlorid als Eluierungsmittel).

## Beispiel 6

Zu einer Lösung von 24,6 Gew. Teilen 2-Chlor-N-chlormethyl-2',6'-dimethylacetanilid und M Gew. Teilen Phasentransferkatalysator in 70 Vol. Teilen Toluol wurde bei 20 bis 25 °C eine Lösung von 7,5 Gew. Teilen Pyrazol und 4,0 Gew. Teilen Natriumhydroxid in 20 Vol. Teilen Wasser unter Turbinieren zugetropft und 3-5 Stunden bei gleicher Temperatur turbiniert. Nach vollständigem Umsatz (DC-Kontrolle) lieferte die organ. Phase nach dreimaligem Waschen mit Wasser, Trocknen und Einengen i. Vac. bei 70 °C Gew. Teile 2-Chlor-2',6-dimethyl-N-(pyrazol-1-yl-methyl)-acetanilid, wie der folgenden Tabelle zu entnehmen ist:

| Versuch Nr. | Phasentransferkatalysator, | M (Gew.Teile Phasentransferkatalysator) | G (Gew.Teile Endprodukt) | Ausbeute in % d.Th., bezogen auf das Ausgangsprodukt Acetanilidderivat | Fp. (°C) | Gehalt (%) (NMR) |
|---|---|---|---|---|---|---|
| 1 | Tetrabutylammoniumjodid | 0,37 | 24,3 | (87,6) | 78 | 98 |
| 2 | Triäthylbenzylammoniumchlorid | 0,20 | 26,1 | (94,3) | 78 | 96 |
| 3 | Tributyl-methylphosphoniumbromid | 0,30 | 25,0 | (90,4) | 77 | 95 |
| 4 | Distearyl-dimethylammoniumchlorid | 0,59 | 25,5 | (92,0) | 78 | 97 |
| 5 | Trimethyl-$C_{13}/C_{15}$-alkylammoniumchlorid | 0,29 | 23,5 | (85,0) | 76 | 96 |
| 6 | Dibenzyl-dimethyl-ammonium-methylsulfat | 0,34 | 25,2 | (91,0) | 76 | 95 |
| 7 | Dimethyl-$C_{12/14}$-alkyl-benzylammoniumchlorid | 0,35 | 24,4 | (88,0) | 78 | 95 |
| 8 | Tetrabutylammonium-hydroxid | 0,26 | 25,2 | (91,0) | 75 | 94 |
| 9 | 18-Krone-6 | 0,26 | 24,8 | (90,0) | 77 | 97 |
| 10+ | Triäthylbenzylammoniumchlorid | 0,05 | 26,2 | (94,6) | 78 | 97 |
| 11+ | Kein Katalysator | - | 25,3 | (91) | 70 | 90 |

+Methylenchlorid anstelle von Toluol

## Beispiel 7

Zu 51,0 Gew. Teilen einer 14,7 proz. wäßrigen Pyrazollösung mit einem Gehalt von 12 bis 13 Prozent Kochsalz, welche durch Spaltung von 1,1,3,3-Tetramethoxypropan mit salzsaurer Hydrazin-Lösung, Verdampfen des gebildeten Methanols im Vakuum und anschließender Neutralisation hergestellt worden war (Org. Prep. Procedd. Int. *5* (1973) 3, 141), wurden 4,0 Gew. Teile Natriumhydroxid und 0,2 Gew. Teile Triäthylbenzylammoniumchlorid zugesetzt. Unter intensivem Rühren wurde dazu eine Lösung von

24,6 Gew. Teilen 2-Chlor-N-chlormethyl-2',6'-dimethyl-acetanilid in 100 Vol. Teilen Toluol bei 20 bis 25 °C in 30 Min. zugetropft und 3 Stunden turbiniert. Aus der Toluolphase isolierte man, wie im Beispiel 6 beschrieben, 23,5 Gew. Teile 2-Chlor-2',6'-dimethyl-(N-pyrazol-1-yl-methyl)-acetanilid vom Fp. 78 °C ($^1$H-NMR-Analyse : 95 %ig).

**Ansprüche**

1. Verfahren zur Herstellung von Acetaniliden der allgemeinen Formel I

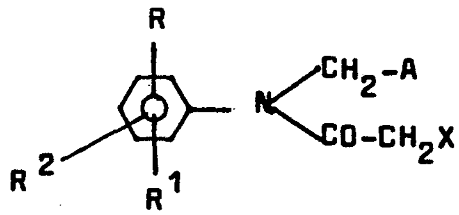

(I)

worin

R Wasserstoff, Alkyl bis zu 5-C-Atomen, Alkoxy bis zu 5-C-Atomen,

$R^1$ Wasserstoff, Halogen, Alkyl bis zu 5-C-Atomen, Alkoxyl bis zu 5-C-Atomen oder Perhalogenalkyl bis zu 5-C-Atomen,

$R^2$ Wasserstoff, Halogen, Alkyl bis zu 5-C-Atomen, Alkoxy bis zu 5-C-Atomen bedeutet oder $R^2$ zusammen mit R eine orthoständig verknüpfte, gegebenenfalls durch Alkylgruppen mit bis zu 4-C-Atomen substituierte Alkylenkette mit bis zu 6-C-Atomen bedeutet,

X Chlor oder Brom,

A ein über ein Ringstickstoffatom gebundenen Azolyl-Rest bedeutet, der einfach oder mehrfach durch Halogen, Phenylreste, Alkyl, Alkoxy, Alkylthio- oder Perfluoralkyl mit jeweils bis zu 4-C-Atomen, Cyan oder Carbalkoxyreste mit bis zu 4-C-Atomen substituiert sein kann, *dadurch gekennzeichnet,* daß man ein 2-Halogen-N-halogenmethylacetanilid der allg. Formel II,

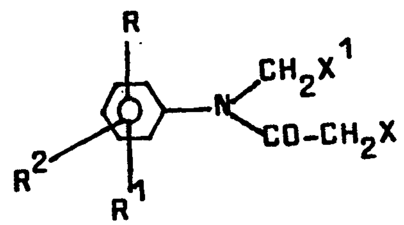

(II)

in welcher R, $R^1$, $R^2$ und X die oben genannten Bedeutungen haben und $X^1$ die gleichen Bedeutungen wie X hat, wobei X und $X^1$ im Einzelfall gleich oder verschieden sind, mit mindestens molaren Mengen eines Azols der Formel H-A, in der A die oben genannten Bedeutungen hat und mindestens molaren Mengen wäßriger Alkalien in einem Zweiphasensystem umsetzt.

2. Verfahren nach Anspruch 1, *dadurch gekennzeichnet,* daß man dem Zweiphasensystem eine als Phasentransferkatalysator geeignete Verbindung aus der Gruppe der Onium-Salze, nämlich der quaternären Ammonium-, Phosphonium- oder Sulfoniumsalze oder der entsprechenden Hydroxide, die auch an feste Träger gebunden sein können, oder der makrocyclischen Heterocyclen, vorzugsweise makrocyclischen Polyäther oder Polyäthylenglykol-diäther oder oberflächenaktiven Substanzen zusetzt.

3. Verfahren nach Anspruch 1, *dadurch gekennzeichnet,* daß man das Azol H-A gelöst oder dispergiert in wäßrigen Alkalien als die eine Phase und eine Verbindung der Formel II gelöst oder dispergiert in einem mit der wäßrigen Phase nicht mischbaren, unter den Reaktionsbedingungen inerten organischen Lösungsmittel als die andere Phase verwendet.

4. Verfahren nach Anspruch 1, *dadurch gekennzeichnet,* daß man die Umsetzung zwischen − 30 ° bis + 100 °C durchführt.

5. Verfahren nach Anspruch 1, *dadurch gekennzeichnet,* daß man als mit Wasser nicht mischbare organische Lösungsmittel aliphatische oder aromatische Kohlenwasserstoffe, Chlorkohlenwasserstoffe, Äther oder Ester verwendet.

**Claims**

1. A process for the preparation of an acetanilide of the general formula I

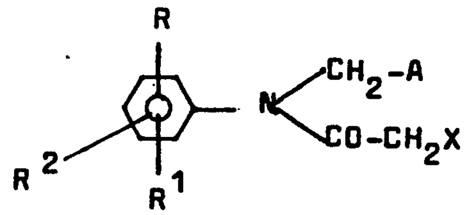

(I)

where

R is hydrogen, alkyl of up to 5 carbon atoms or alkoxy of up to 5 carbon atoms,

$R^1$ is hydrogen, halogen, alkyl of up to 5 carbon atoms, alkoxy of up to 5 carbon atoms or perhaloalkyl of up to 5 carbon atoms,

$R^2$ is hydrogen, halogen, alkyl of up to 5 carbon atoms or alkoxy of up to 5 carbon atoms, or $R^2$ together with R is alkylene of up to 6 carbon atoms, which may or may not be substituted by alkyl of up to 4 carbon atoms, and is ortho-bonded to the benzene ring,

X is chlorine or bromine, and

A is an azolyl radical which is bonded via a ring nitrogen and may be monosubstituted or polysubstituted by halogen, phenyl, alkyl, alkoxy, alkylthio or perfluoroalkyl each of up to 4 carbon atoms, cyano or carboalkoxy of up to 4 carbon atoms, *characterized in that* a 2-halo-N-halomethylacetanilide of the general formula II

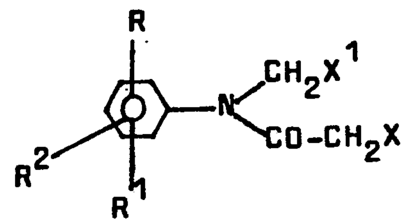

(II).

where R, $R^1$, $R^2$ and X have the above meanings and $X^1$ has the same meanings as X, and X and $X^1$ may be identical or different, is reacted with an at least molar amount of an azole of the formula H-A, where A has the above meanings, in the presence of an at least molar amount of an aqueous alkali, in a two-phase system.

2. A process as claimed in claim 1, *characterized in that* there is added to the two-phase system a compound suitable as phase transfer catalyst and selected from the group consisting of an onium salt, namely a quaternary ammonium, phosphonium or sulfonium salt, a corresponding hydroxide, which compounds may also be bonded to a solid carrier, a macrocyclic heterocyclic compound, preferably a macrocyclic polyether, a polyethylene glycol diether and a surfactant.

3. A process as claimed in claim 1, *characterized in that* a solution or dispersion of the azole H-A in an aqueous alkali is used as one phase and a solution or disperson of a compound of the formula II in an organic solvent which is inert under the reaction conditions and is immiscible with the aqueous phase is used as the other phase.

4. A process as claimed in claim 1, *characterized in that* the reaction is carried out at from – 30 ° to + 100 °C.

5. A process as claimed in claim 1, *characterized in that* an aliphatic or aromatic hydrocarbon, chlorohydrocarbon, ether or ester is used as the water-immiscible organic solvent.

**Revendications**

1. Procédé de préparation d'acétanilides de formule générale I

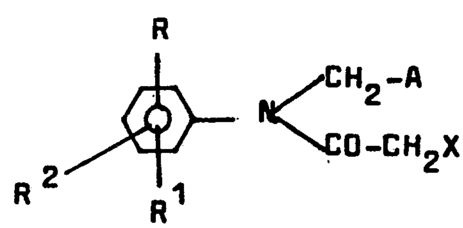

(I)

dans laquelle

R représente hydrogène, alkyle ayant jusqu'à 5 atomes C, alcoxy ayant jusqu'à 5 atomes C,

$R^1$ est hydrogène, halogène, alkyle ayant jusqu'à 5 atomes C, alcoxy ayant jusqu'à 5 atomes C ou perhalogènealkyle ayant jusqu'à 5 atomes C,

$R^2$ est hydrogène, halogène, alkyle jusqu'à 5 atomes C, alcoxy ayant jusqu'à 5 atomes C ou $R^2$ représente avec R une chaîne alkylène ayant jusqu'à 6 atomes C, reliée en position ortho, éventuellement substituée par des groupes alkyle ayant jusqu'à 4 atomes C,

X est du chlore ou du brome,

A est un reste azolyle lié par l'intermédiaire d'un atome d'azote du noyau, reste qui peut être substitué une ou plusieurs fois par un halogène, un reste phényle, alkyle, alcoxy, alkylthio ou perfluoroalkyle ayant jusqu'à 4 atomes C, un reste cyano ou carbalcoxy ayant jusqu'à 4 atomes C, caractérisé par le fait que l'on fait réagir avec au moins des quantités molaires d'un azol de formule H-A, dans laquelle A a la signification indiquée plus haut, et au moins des quantités molaires d'alcalis aqueux, dans un système à deux phases, un 2-halogéno-N-halogénométhylacétanilide de formule générale II

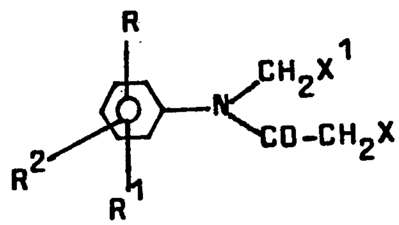

(II)

dans laquelle R, $R^1$, $R^2$ et X ont les significations indiquées plus haut et $X^1$ a la même signification que X, X et $X^1$ étant identiques ou différents.

2. Procédé selon la revendication 1, caractérisé par le fait que l'on ajoute au système à deux phases un composé convenant comme catalyseur de transfert de phases et appartenant au groupe des sels onium, notamment les sels d'ammonium, phosphonium ou sulfonium quaternaires ou leurs hydroxydes correspondants qui peuvent aussi être liés à des supports solides, ou des hétérocycles macrocycliques, de préférence des polyéthers ou polyéthylène-glycol-diéthers macroxycliques, ou des substances tensioactives.

3. Procédé selon la revendication 1, caractérisé par le fait que l'on utilise comme l'une des phases, l'azol-H-A dissous ou dispersé dans des alcalis aqueux et comme l'autre phase, un composé de formule II dissous ou dispersé dans un solvant organique, inerte dans les conditions de réaction et non miscible avec la phase aqueuse.

4. Procédé selon la revendication 1, caractérisé par le fait que l'on effectue la réaction entre − 30 ° et + 100 °C.

5. Procédé selon la revendication 1, caractérisé par le fait que l'on utilise comme solvant organique non miscible avec l'eau, un hydrocarbure aliphatique ou aromatique, un hydrocarbure chloré, un éther ou un ester.